# EUROPEAN PATENT APPLICATION

(11) **EP 3 821 782 A1**
(43) Date of publication of application: **19.05.2021**
(21) Application number: 19208950.6
(22) Date of filing: 13.11.2019
(51) Int. Cl.: A61B 1/00, A61B 1/018, A61B 1/227

(54) **MEDICAL DEVICE**

(71) Applicant: Biovisor AB, 11437 Stockholm (SE)
(72) Inventor: Bengtsson, Andreas, 031 89 Orihuela Costa (ES)
(74) Representative: Groth & Co. KB

(57) **Abstract**

The present invention relates to a speculum (1) for use in conjunction with an instrument for inspecting and treating a human or animal body orifice, wherein the speculum (1) is provided with
i) a first channel (2) being substantially centrally aligned and extending substantially along length L of the speculum (1) for visual control; and
ii) at least one second channel (3) adapted for insertion of a tool for treatment of a body orifice extending along the outer wall and/or the inner wall of the speculum (1).

The invention also relates to a method for inspecting and treating a body orifice by means of speculum (1) in conjunction with an instrument for inspection and treatment, and the use of the speculum (1).

## Description

The present invention relates to a speculum for use in conjunction with an instrument for inspecting and treating a human or animal body orifice. The invention also relates to a method of inspecting and treating a human or animal by means of the speculum in conjunction with the instrument. The invention also relates to the use of the speculum for inspecting and treating inter alia ears or noses.

### Background of the invention

Specula are well-known medical tools used in conjunction with e.g. otoscopes or other devices for investigating body orifices, with a form dependent on the orifice for which it is designed. A speculum is arranged on an investigation instrument such as an otoscope which is a medical device allowing for inspection of e.g. an ear canal and tympanic membrane (eardrum). Otoscopes may also be used to examine patients' noses and other orifices.

Screening for illnesses such as otitis media and otitis externa, infections of the middle and outer parts of the ear and investigation of other ear symptoms can thereby be performed. An otoscope may also be used to remove the presence of cerumen (earwax), bulging, infection, shed skin, pus, canal skin edema, or a foreign object.

The most commonly used otoscopes consist of a handle and a head. The head contains a light source and a simple low-power magnifying lens, typically around 8 diopters (3.00x Mag). The distal (front) end of the otoscope has an attachment for specula, typically disposable plastic ear specula. An examiner can then look through a lens of the instrument and see inside the ear canal. In many models, the lens can be removed, which allows the examiner to insert instruments, e.g. suction tips or other actuation elements through the otoscope into the ear canal, such as for removing earwax (cerumen).

However, the sight is reduced as the lens is withdrawn and the instrument itself may occlude the sight of the examiner. The same may apply to other body orifices. There is thus a need to improve the optical view of an examiner while inspecting and treating a body orifice. The present invention intends to provide an otoscope, rhinoscope or other device with improved optical control while treating an orifice.

### The invention

The present invention relates to a speculum for use in conjunction with an instrument for inspecting and treating a human or animal body orifice, wherein the speculum is provided with
i) a first channel being substantially centrally aligned and extending substantially along length L of the speculum for visual control; and
ii) at least one second channel adapted for insertion of a tool for treatment of a body orifice extending along the outer wall and/or the inner wall of the speculum.

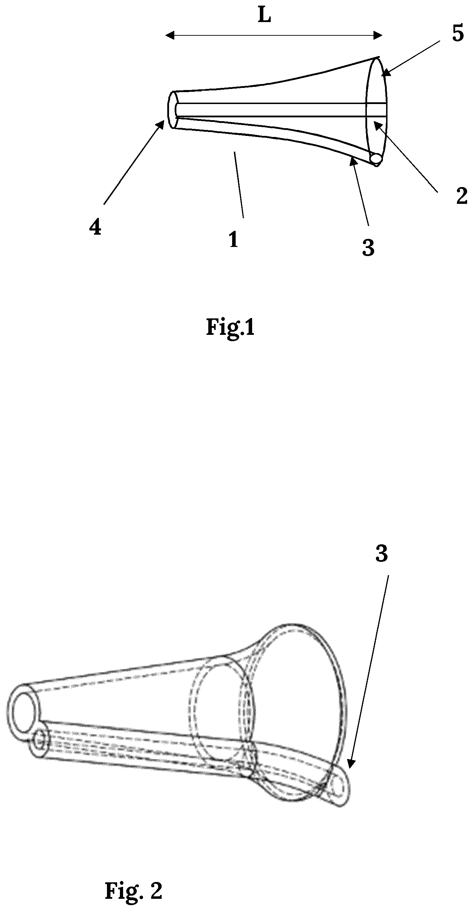

According to one embodiment, said at least one second channel extends outside the speculum wall at its proximal end, i.e. at the end of the examiner (i.e. end 5 in fig.1), and extends inside the speculum wall at its distal end (i.e. end 4 in fig.1).

Such embodiment may be arranged by providing a hole in the speculum wall through which the second channel can pass, e.g. at the midpart thereof or in the vicinity of the midpart of its axial length. According to another embodiment, the portion of the second channel may extend along the inner wall of the speculum at the end closest to the examiner and at the outer wall of the speculum at the end facing away from the examiner. According to one embodiment, the entire at least second channel may also extend only along the inner or outer wall of the speculum.

According to one embodiment, the first and the at least second channel is an integral part of the speculum.

According to one embodiment, at least second channel has an inner diameter ranging from 1 to 5 mm, e.g. 1 to 3 mm.

According to one embodiment, the length of said at least second channel ranges from 25 to 120 mm, for example 25 to 70 mm, such as from 25 to 50 mm. According to one embodiment, the length of said at least one second channel ranges from 70 to 120 mm, for example 90 to 120 mm.

According to one embodiment, the inner diameter of said at least one second channel varies from 1 to 5 mm along its extension, for example from 1 to 3 mm, or 1 to 2 mm.

According to one embodiment, the first channel has an inner diameter ranging from 1.5 to 20 mm. According to one embodiment, the first channel has an inner diameter ranging from 1.5 to 10 mm, for example 1.5 to 5 mm or from 1.5 to 3 mm.

According to one embodiment, the first channel has an inner diameter ranging from 10 to 20 mm, for example 15 to 20 mm.

According to one embodiment, the length of said first channel ranges from 80 to 100% of the length of the speculum, for example 90 to 100 % of the length of the speculum.

According to one embodiment, the thickness of the first and said at least one second channel ranges from 0.2 mm to 1 mm.

According to one embodiment, the entire speculum is one integral part. According to one embodiment, a camera is arranged to provide for optical control through said first channel.

According to one embodiment, said at least one second channel extends on the outer wall at its distal end and at the inner wall at its proximal end.

The invention also relates to a method for inspecting and treating a body orifice by means of a speculum as described herein in conjunction with an instrument for inspection and treatment, wherein the speculum is inserted in a body orifice, and wherein optical control means are provided which safeguard optical control through said first channel and at least one tool is inserted in said body orifice via said at least one second channel for treating said body orifice.

The invention also relates to the use of a speculum as described herein for inspecting and treating the ear. The invention also relates to the use of a speculum as described herein for inspecting and treating an animal.

By the term "treating" is meant any surgical procedure or other procedure such as removing cerumen (earwax), treating or inspecting bulging, infection, shed skin, pus, canal skin edema, removing a foreign object, or taking a biopsy.

According to one embodiment, a second tool is inserted in the same second channel to treat a body orifice. According to one embodiment, a third channel may be provided which may extend along the speculum wall, such as an outer wall or inner wall of the speculum. According to one embodiment, a further tool may be inserted via said third channel. According to one embodiment, inspection and treatment of a body orifice is performed by inserting a tool via both the second and the third channel. The tool may be the same or different tools.

The speculum may be releasably attached to the inspection instrument such as an otoscope or rhinoscope. The speculum may be attached to the instrument in any conventional manner known in the art.

Preferably, the inspection instrument such as an otoscope comprises a light source and an optical imaging system. The optical imaging system can preferably focus an optical image of a target viewed through the speculum inserted in e.g. the ear canal. The dimension and shape of the speculum may vary depending on the orifice or whether a human or a specific animal is to be examined. Preferably, the speculum has a substantially hollow conical shape, or a substantially cylindrical portion and a conical portion, wherein the cylindrical portion is coaxial with the conical portion.

Whereas the term "otoscope" is primarily contemplated to be used in conjunction with the speculum, the speculum may likewise be used with any other examination instrument such as a rhinoscope. Thus, what is stated herein with respect to "otoscope" is applicable to any other examination instrument.

According to one embodiment, the otoscope is a monocular or binocular otoscope, preferably a binocular otoscope. A binocular device imparts a three dimensional view of the ear canal, rather than a two-dimensional view of the ear canal obtained by a monocular otoscope. Preferably, a 3D otoscope is provided, preferably with a metal ear speculum, providing a larger field of view and depth perception.

According to one embodiment, a digital video otoscope may be used in conjunction with the speculum. Such an otoscope may be provided with a camera, preferably arranged in said first centrally arranged channel. According to one embodiment, the video otoscope includes a power supply and lighting unit for transmission of power and light inputs to the otoscope and a video peripheral device such as a video monitor for displaying a visual image of e.g. a target object such as an object which shall be removed from the ear or area undergoing inspection and treatment.

According to one embodiment, a 3D printer may be used to provide the speculum including the channels as described herein.

The invention may be used for a variety of human and animal body orifices or cavities such as medical inspection and intervention or treatment of the nose or ear.

### Brief description of the drawings

Figure 1 shows an overview of the speculum with a channel extending along the speculum wall on the inside thereof. Figure 2 shows an overview of a speculum with a channel extending outside the speculum wall.

### Detailed description of the invention

Figure 1 shows a speculum 1 which may be used in combination with an otoscope, rhinoscope or similar examining instrument for examining human or animal body orifices such as the ear or the nose. In the following an otoscope connected to the spectrum will illustrate the invention for examination of an ear. The speculum may, however, be used in any other human or animal body orifice.

The otoscope (not shown) is arranged to the speculum 1 at the proximal end 5 thereof. The distal end 4 of the speculum is inserted in the ear canal for examination. The otoscope comprises a lens which imparts a magnified optical view through the central channel 2 of the spectrum. While an examiner has a good magnified view through central channel 2 by means of the otoscope, a tool (not shown) such as a suction device, e.g. suction tips, or means for removing an extraneous object may be inserted through channel 3 along the inner wall of the speculum. Figure 2 shows a similar speculum but in which the channel 3 is extending on the outside of the speculum wall. The central channel 2 is not shown in figure 2.

## Claims

1. Speculum (1) for use in conjunction with an instrument for inspecting and treating a human or animal body orifice, wherein the speculum (1) is provided with
iii) a first channel (2) being substantially centrally aligned and extending substantially along length L of the speculum (1) for visual control; and
iv) at least one second channel (3) adapted for insertion of a tool for treatment of a body orifice extending along the outer wall and/or the inner wall of the speculum (1).

2. Speculum (1) according to claim 1, wherein said first channel (2) and said at least second channel (3) is an integral part of the speculum (1).

3. Speculum (1) according to claim 1 or 2, wherein said at least second channel (3) has an inner diameter ranging from 1 to 5 mm.

4. Speculum (1) according to any one of claims 1 to 3, wherein the length of the second channel (3) ranges from 25 to 120 mm.

5. Speculum (1) according to any one of claims 1 to 4, wherein the inner diameter of the second channel (3) varies from 1 to 5 mm along its extension.

6. Speculum (1) according to any one of claims 1 to 5, wherein said first channel (2) has an inner diameter ranging from 1.5 to 20 mm.

7. Speculum (1) according to any one of claims 1 to 6, wherein the length of said first channel (2) ranges from 80 to 100% of the length of the speculum.

8. Speculum (1) according to any one of claims 1 to 7, wherein the ratio of the inner diameter of said first channel (2) to the inner diameter of said at least second channel (3) ranges from 1 to 2.5.

9. Speculum (1) according to any one of claims 1 to 8, wherein the thickness of the first (2) and said at least one second (3) channel ranges from 0.2 mm to 1 mm.

10. Speculum (1) according to any one of claims 1 to 9, wherein the entire speculum (1) is one integral part.

11. Speculum (1) according to any one of claims 1 to 10, wherein a camera is arranged to provide for optical control through said first channel (2).

12. Speculum (1) according to any one of claims 1 to 11, wherein said at least one second channel (3) extends on the outer wall of the speculum (1) at its distal end (4) and at the inner wall of the speculum (1) at its proximal end (5).

13. Method for inspecting and treating a body orifice by means of a speculum (1) as claimed in any of claims 1 to 12 in conjunction with an instrument for inspection and treatment, wherein the speculum (1) is inserted in a body orifice, and wherein optical control means are provided which safeguard optical control through said first channel (2) and at least one tool is inserted in said body orifice via said at least one second channel (3) for treating said body orifice.

14. Use of a speculum (1) according to any one of claims 1 to 12 for inspecting and treating the ear.

15. Use of a speculum (1) according to any one of claims 1 to 12 for inspecting and treating an animal.
